# EUROPÄISCHE PATENTSCHRIFT

(11) **EP 0 833 810 B1**
(45) Veröffentlichungstag und Bekanntmachung des Hinweises auf die Patenterteilung: **09.02.2000**
(21) Anmeldenummer: 96922000.3
(22) Anmeldetag: 17.06.1996
(51) Int. Cl.: C07C 53/48, C07C 51/58

(54) **HERSTELLUNG VON DIFLUORCHLOR- UND DICHLORACETYLCHLORID**
DIFLUOROCHLORACETYL CHLORIDE AND DICHLORACETYL CHLORIDE PREPARATION
PREPARATION DE CHLORURE DE DIFLUOROCHLORACETYLE ET DE DICHLORACETYLE

(30) Priorität: 23.06.1995 DE 19522533; 17.05.1996 DE 19620018
(43) Veröffentlichungstag der Anmeldung: 08.04.1998
(73) Patentinhaber: Solvay Fluor und Derivate GmbH, D-30173 Hannover (DE)
(72) Erfinder: BRAUN, Max, D-30900 Wedemark (DE); RUDOLPH, Werner, D-30559 Hannover (DE); EICHHOLZ, Kerstin, D-30855 Langenhagen (DE)
(74) Vertreter: Lauer, Dieter, Dr.
(86) Internationale Anmeldenummer: EP9602596
(87) Internationale Veröffentlichungsnummer: WO9700847

(56) Entgegenhaltungen:
- DE-A- 2 118 540
- DE-B- 1 069 137
- US-A- 2 736 695

## Beschreibung

Die Erfindung bezieht sich auf ein Verfahren zur Herstellung von Difluorchlor- und Dichloracetylchlorid durch photochemische Oxidation.

Dichloracetylchlorid ist Zwischenprodukt in der chemischen Synthese. Beispielsweise können die in der internationalen Anmeldung WO 93/24483 (Chemical Abstracts 120, 1994, Referat 245064y) beschriebenen Herbizide, bei denen es sich um bestimmte Pyrrolidon-Verbindungen handelt, unter Verwendung von Dichloracetylchlorid synthetisiert werden.

Die Herstellung von Di- bzw. Trichloracetylchlorid durch photochemische Oxidation von Trichlorethylen bzw. Tetrachlorethylen mit Sauerstoff in Gegenwart von Chlor unter Bestrahlung mit kurzwelligem Licht in flüssiger Phase ist aus der Deutschen Patentschrift 759 963 bekannt. Die Oxidation von Tetrachlorethylen durch Quarzglas in Anwesenheit von Chlor unter Bestrahlung mit Licht einer Wellenlänge von 436 nm (= 436 mµ) unter Bildung von CCl₃ C(O) Cl wird von Schott und Schumacher in Z. Phys. Chem. 49 (1941), Seiten 107 bis 125 beschrieben, die entsprechende Oxidation von Trichlorethylen in Z. Phys. Chem. Abt. B, 37 (1937), Seiten 365 bis 373. Gemäß jener Patentschrift soll die Photooxidation in der Gasphase jedoch infolge der hohen Wärmeentwicklung und der Schwierigkeit der Abführung der Wärme aus den gasförmigen Produkten nur in kleinstem Maßstab durchführbar sein; in der Folge wendete sich (schon beim Verfahren des deutschen Patents DE 759 963) die Fachwelt der Flüssigphasenoxidation zu. Wie in der Europäischen Patentschrift EP-B-0 422 520 offenbart wird, entstehen bei der Flüssigphasenoxidation jedoch in erhöhtem Maße Epoxide. Gemäß der Europäischen Patentschrift wird das Ausgangsprodukt in einem dünnen Film durch die Reaktionszone geführt. Eine weitere Verbesserung des Flüssigphasenverfahrens wird in der Europäischen Patentanmeldung EP-A-0 623 578 angestrebt. Bei jenem Verfahren, das die Umsetzung von Trichlorethylen mit Sauerstoff in flüssiger Phase in Abwesenheit von Chlor unter Bestrahlung mit kurzwelligem Licht vorsieht, werden bestimmte stickstoffhaltige Basen (sekundäre Amine) zugesetzt, um die Umwandlung der nebenbei produzierten Epoxide in Dichloracetylchlorid zu beschleunigen. Durch die Zugabe eines solchen Katalysators, der naturgemäß aus dem Reaktionsgemisch oder Produkt wieder abgetrennt werden muß, wird das Verfahren stark verkompliziert.

Chlordifluoracetylchlorid ist ebenfalls Zwischenprodukt in der chemischen Synthese, beispielsweise in der Farbstoffherstellung. Alkyl- und Arylhalogenide können durch ein Derivat von Chlordifluoracetylchlorid, nämlich durch den Methylester, in Anwesenheit von Kaliumfluorid und Kupferjodid trifluormethyliert werden. Der erwähnte Methylester ist auch Vorprodukt für die Herstellung von Difluorcarben, siehe G. A. Wheaton und D. J. Donald in J. Fluorine Chem. 8 (1976), Seiten 97 bis 100. Difluorcarben wird bei der Insektizid-Herstellung eingesetzt, siehe EP-A 198 791 (US-A 4,701,563). Die Herstellung von Difluorcarben aus Trifluormethyl-Phenylquecksilber und anderen Verbindungen dieser Art ist aus Umweltgesichtspunkten problematisch.

Die deutsche Offenlegungsschrift DE 43 42 601 A1 offenbart die Herstellung von u. a. Chlordifluoracetylchlorid durch photochemische Oxidation von 1,1-Difluor-1,2,2-trichlorethan unter aktivierender Bestrahlung in der Gasphase ohne Zusatz von Sensibilisatoren. Die deutsche Offenlegungsschrift DE-OS 44 20 763 A1 offenbart ein entsprechendes Verfahren unter Zusatz von elementarem Chlor als Sensibilisator und Bestrahlung mit Licht einer Wellenlänge λ ≥ 290 nm. Die deutsche Auslegeschrift DE-AS 1 069 137 offenbart u. a. die Herstellung von Chlordifluoracetylchlorid aus 1,1-Difluor-2,2-dichlorethylen oder aus 1,1-Difluor-1,2,2-trichlorethan oder 1,1-Difluor-2,2,2-trichlorethan durch photochemische Oxidation. Sofern man von den gesättigten Ethanderivaten ausgeht, soll man zweckmäßig bei dieser Ausführungsform in Gegenwart von Chlor arbeiten.

Die DE-A 21 18 540 offenbart ein Verfahren zur Herstellung von Trichloracetylchlorid mittels photochemisch sensibilisierter Gasphasenoxidation von Perchlorethylen (CCl₂ = CCl₂) mit Sauerstoff. Um die Oxidation in der Gasphase unter gut gesteuerten Temperaturbedingungen durchzuführen, wird ein flüssiges Kühlmittel kontinuierlich in Tropfenform in das Reaktionsgefäß eingeführt. Das Kühlmittel verdampft und führt dabei die Wärme ab. Als Kühlmittel kann man Perchlorethylen, Trichloracetylchlorid oder inerte Stoffe wie CCl₄ oder Pentachlorethan einsetzen.

Aufgabe der vorliegenden Erfindung ist es, ein Verfahren zur Herstellung von Difluorchlor- und Dichloracetylchlorid durch photochemische Oxidation anzugeben, mit welchem selektiv, insbesondere im Hinblick auf die Unterdrückung der Epoxidbildung, und in akzeptabler Ausbeute die gewünschten Produkte in einem technisch einfachen Verfahren hergestellt werden können. Diese Aufgabe wird durch das Verfahren der vorliegenden Erfindung gelöst.

Erfindungsgemäß stellt man in α-Stellung durch mindestens 1 Chloratom substituierte Carbonsäurechloride, nämlich Dichloracetylchlorid durch photochemische Oxidation von CCl₂ = CHCl oder CHCl₂CHCl₂, oder Difluorchloracetylchlorid durch photochemische Oxidation von CF₂ = CCl₂ als C2-Reaktant mit Sauerstoff als Reaktant in der Gasphase her, wobei man den C2-Reaktanten dampfförmig in den Reaktor einbringt, man das Verfahren kontinuierlich durchführt, man in Anwesenheit von elementarem Chlor arbeitet, wobei man eine aktivierende Bestrahlung mit Licht einer Wellenlänge von λ ≥ 280 nm vornimmt, und die Reaktanten in der Gasphase miteinander umsetzt.

Zur Bestrahlung kann man vorteilhaft Bestrahlungslampen (z. B. Philips-Leuchtstoff-Röhren) verwenden, die nur (UV-)Licht einer Wellenlänge bei oder oberhalb von 280 nm (λ ≥ 280 nm) abstrahlen. Hier ist die Bestrahlung durch Quarzglas möglich. Einzige Voraussetzung für diese Variante ist, daß diese Lampen im Absorptionsbereich des elementaren Chlor emittieren. Alternativ dazu kann man auch beispielsweise Bestrahlungslampen (z. B. Hg-Mittel- oder Hochdruckstrahler) verwenden, die auch einige Linien im Bereich unterhalb von 280 nm (λ < 280 nm) emittieren. Bei dieser Variante muß durch ein Glas bestrahlt werden, das nur für Licht einer Wellenlänge von 280 nm oder darüber (λ ≥ 280 nm) durchlässig ist, also den kürzerwelligen Bestrahlungsanteil mit λ < 280 nm herausfiltert. Gut geeignet dafür sind beispielsweise Borosilikat-Gläser. Derartige Gläser enthalten üblicherweise 7 bis 13 % B₂O₃, 70 bis 80 % SiO₂, ferner 2 bis 7 % Al₂O₃ und 4 bis 8 % Na₂O + K₂O sowie 0 bis 5 % Erdalkalimetalloxide. Bekannte Warenzeichen für Borosilikat-Gläser sind Duran, Pyrex und Solidex. Selbstverständlich kann man auch so vorgehen, daß man einerseits eine Bestrahlungslampe einsetzt, die Licht oberhalb der angegebenen Wellenlänge abstrahlt, und zusätzlich Gläser verwendet, die für Licht oberhalb der angegebenen Wellenlänge durchlässig sind (d.h. für Licht unterhalb der angegebenen Wellenlänge entsprechend undurchlässig sind).

Gut geeignet zur Bestrahlung sind auch Lampen, z. B. Hg-Hochdrucklampen, die aufgrund eines Dotierungsmittel überwiegend oder nur im Wellenbereich bei oder oberhalb von 280 nm abstrahlen. Hg-Hochdruckstrahler beispielsweise weisen eine recht intensive Bande im Bereich von 254 nm auf, die, wie oben beschrieben worden ist, z. B. durch Borosilikat-Glas herausgefiltert wird. Bei durch Metalliodide dotierten Hg-Hochdruckstrahlern ist diese Linie stark unterdrückt. Überraschend ist die oft überproportionale Erhöhung der Umsatzrate bei solchen dotierten Strahlern. Hervorragende Ergebnisse im Hinblick auf Umsatzrate und Selektivität erzielt man mit Hg-Hochdruckstrahlern, die mit Galliumiodid dotiert sind, und besonders mit Strahlern, die mit Thalliumiodid oder Cadmiumiodid dotiert sind. Auch bei Verwendung solcher Strahler setzt man vorteilhaft Glas ein, das den kürzerwelligen Strahlungsanteil mit λ < 280 nm herausfiltert. Zweckmäßig und technisch vorteilhaft ist es, den gesamten Strahlungsbereich mit Wellenlängen oberhalb der genannten Grenze auszunutzen.

In bezug auf Umsetzungstemperatur und Druck kann man derart arbeiten, daß es zu keiner Kondensation innerhalb des Photoreaktors kommt. Vorzugsweise führt man die Umsetzung bei Temperaturen bis zu 200 °C durch. Die Herstellung von Dichlor- und Chlordifluoracetylchlorid führt man insbesondere in einem Temperaturbereich von 50 bis 150 °C durch. Man kann bei vermindertem Druck arbeiten, vorzugsweise beträgt er mindestens 1 bar absolut. Insbesondere arbeitet man bei einem Druck von 1 bis 10 atm (abs.) vorzugsweise 1 bis 3 atm (abs.). Ganz besonders bevorzugt arbeitet man drucklos. Der Begriff "drucklos" bedeutet im Rahmen der vorliegenden Erfindung, daß auf die Reaktionsmischung außer dem Umgebungsdruck (d. h. etwa 1 atm), dem Förderdruck des Sauerstoffgases (bzw. des sauerstoffhaltigen Gases, man kann z. B. Luft einsetzen) und des Chlors und dem sich gegebenenfalls ausbildenden Druck durch bei der Reaktion entstehendes Chlorwasserstoffgas kein zusätzlicher Druck einwirkt. Der Gesamtdruck im Reaktor ist dann zweckmäßig kleiner als 2 bar absolut, je nach Förderdruck sogar kleiner als 1,5 bar absolut, aber größer als Umgebungsdruck.

Als besonders vorteilhaft hat es sich erwiesen, in bezug auf Umsetzungstemperatur und Druck so zu arbeiten, daß die C2-Ausgangsverbindung gasförmig (bzw. dampfförmig) im Reaktor vorliegt, das Reaktionsprodukt jedoch kondensiert. Die Bildung von Phosgen wird hierbei unterdrückt, die Selektivität wird erhöht. Bei der Herstellung von Dichloracetylchlorid aus Trichlorethylen arbeitet man z. B. zwischen 80 und 110 °C (der Druck liegt dann z. B. zwischen 1 und 2 bar absolut).

Zweckmäßig zieht man das Reaktionsprodukt in dieser Variante flüssig vom Boden der Bestrahlungsapparatur ab, die gasförmigen Ausgangsverbindungen (O₂, Chlor, C2-Reaktant) speist man separat oder als Gemisch weiter oben in Richtung auf den Kopf der Bestrahlungsapparatur ein oder am Kopf selbst.

Das Verfahren fürht man vorteilhaft in einer Durchflußapparatur durch. Man geht so vor, daß man kontinuierlich Ausgangsmaterial (die entsprechende Wasserstoff und Halogen enthaltende Ausgangsverbindung, Chlor und Sauerstoff) in die Durchflußapparatur einspeist und entsprechend der eingespeisten Menge kontinuierlich Reaktionsprodukt abzieht.

Das Molverhältnis zwischen dem C2-Reaktanten und dem elementaren Chlor kann in einem weiten Bereich schwanken z. B. von 1:0,01 bis 1:1. Besonders gute Ergebnisse werden erzielt, wenn das Molverhältnis zwischen der Ausgangsverbindung und dem elementaren Chlor im Bereich von 1:0,08 bis 1:0,2 liegt.

Das Molverhältnis zwischen dem C2-Reaktanten und Sauerstoff kann ebenfalls in einem weiten Bereich schwanken. Zweckmäßig setzt man mindestens 0,3 Mol Sauerstoff pro Mol Ausgangsverbindung ein. Besonders gute Ergebnisse werden erzielt, wenn das Molverhältnis zwischen der Ausgangsverbindung und dem Sauerstoff im Bereich von 1:0,3 bis 1:5, vorzugsweise 1:0,4 bis 1:5, insbesondere 1:0,4 bis 1:1,8 ganz besonders 1:0,5 bis 1:1,8 liegt. Sehr gute Ergebnisse werden schon erhalten, wenn das Molverhältnis von Ausgangsverbindung und Sauerstoff zwischen 1:0,5 und 1:1,1 liegt. Der Sauerstoff kann in Form von Luft oder als 0₂/Inertgas-Gemisch, vorzugsweise aber als reiner Sauerstoff eingesetzt werden.

Die aktivierende Bestrahlung wird vorzugsweise mit Strahlern durchgeführt, die Licht abgeben, das mindestens teilweise im UV-Bereich liegt. Beispielsweise sind Hg-Hochdruck-und Mitteldruckstrahler geeignet. Auch Leuchtstoffröhren, z. B. Philips-Leuchtstoffröhren mit selektiver Emission bei 350 nm, sind einsetzbar. Als Werkstoff für die entsprechenden Apparaturbestandteile empfiehlt sich UV-durchlässiges Material. Quarz kann, wie gesagt, verwendet werden, sofern die Strahler Licht bei oder oberhalb einer Wellenlänge von 280 nm abstrahlen. Anderenfalls oder alternativ setzt man die genannten Borosilikat-Gläser ein.

In bezug auf die Produktreinheit ist es wünschenswert, daß möglichst wenig Wasser bei der Umsetzung vorhanden ist. Gewünschtenfalls kann man die Reaktanten in bekannter Weise von mitgeschlepptem Wasser befreien, z. B. mittels Molekularsieb.

Die durchschnittliche Verweilzeit im Reaktionsgefäß liegt vorzugsweise zwischen 0,01 und 30 Minuten, vorzugsweise zwischen 0,01 und 3 Minuten, insbesondere zwischen 0,5 und 3,0 Minuten. Selbst bei sehr kurzer Verweilzeiten, z. B. zwischen 0,04 und 0,5 Minuten werden schon gute Ergebnisse erzielt. Die optimale durchschnittliche Verweilzeit, die u. a. von der Lampenleistung und von geometrischen Parametern der Bestrahlungsapparatur (Durchflußapparatur) abhängig ist, kann man durch einfache Handversuche und Analyse des Produktstromes, z. B. durch Gaschromatographie, ermitteln.

Bessere Umsatzraten und höhere Selektivität können erzielt werden, wenn statt einer einzelnen Bestrahlungslampe mit bestimmter Leistung zwei oder mehr leistungsschwächere Lampen gleicher Gesamtleistung in hintereinandergeschalteten Reaktoren eingesetzt werden. Dabei wird das Produkt zweckmäßig nach Verlassen der jeweiligen Reaktionen abgetrennt, z. B. durch Ausfrieren. Auch ist eine gute Verwirbelung der Reaktionsmischung, z. B. durch geeignete Einbauten im Reaktor, oft von Vorteil.

Das Verfahren bietet einige überraschende Vorteile. Überraschend ist auch die hohe Umsatzrate bei hoher Selektivität trotz des bevorzugten Einsatzes von elementarem Chlor (man findet keine bzw. lediglich Spuren chlorierter Nebenprodukte), insbesondere bei Anwendung von mit Metalliodid dotierten Hg-Hochdruckstrahlern. Das erfindungsgemäße Verfahren weist u. a. folgende Vorteile auf: es ist technisch einfach; es ist selektiv, nur geringe Mengen an Epoxiden und Phosgen werden gebildet; es wird kein Katalysator benötigt; es kann kontinuierlich durchgeführt werden, gewünschtenfalls auch drucklos; die Quantenausbeute ist sehr hoch; die Umsetzungsgeschwindigkeit ist sehr hoch.

Die folgenden Beispiele sollen die Erfindung weiter erläutern, ohne sie in ihrem Umfang einzuschränken. Man arbeitet unter Beachtung der für das Arbeiten mit molekularem O₂ notwendigen Sicherheitsvorkehrungen.

### Beispiel 1:

### Kontinuierliche Herstellung von Dichloracetylchlorid (DCAC) aus 1,1,2,2-Tetrachlorethan (TCE) durch Borosilikat-Glas und Cl₂-Sensibilisierung

In einem 400 ml Photoreaktor aus Pyrex-Glas mit einem mit CdI₂-dotiertem Hg-Hochdruckstrahler (Bezeichnung: TQ 718 Z 3, Firma Heraeus Noblelight) wurde eine Mischung aus 1,1,2,2-Tetrachlorethan (aus Vorverdampfer mit T = 200 °C), Sauerstoff und Chlor im molaren Verhältnis 1:1,1:0,1 gasförmig von unten zugeführt und mit einer Lampenleistung von 700 W bei einer Reaktorinnentemperatur von 170 °C durch Pyrex-Glas mit λ > 280 nm bestrahlt. Die Dosierung betrug 2 mol in der Stunde. Der den Reaktor verlassende gasförmige Produktstrom enthielt DCAC in einer Selektivität von 79 %, der Umsatz betrug 83 %.

### Beispiel 2:

### Kontinuierliche Herstellung von Dichloracetylchlorid (DCAC) durch Photooxidation von Trichlorethylen durch Borosilikat-Glas und Cl₂-Sensibilisierung in der Gasphase bei 90 °C

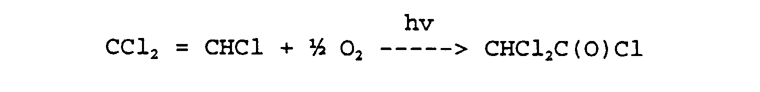

Einem 400 ml Photolyse Tauchschachtreaktor aus Pyrex-Glas (außen mit Doppelmantel für die Heizung) ausgestattet mit einem nicht dotierten Hg-Hochdruckstrahler (Bezeichnung: TQ 718, Firma Heraeus Noblelight) wurde eine Mischung aus Trichlorethylen (aus Vorverdampfer mit T = 150 °C), Sauerstoff und Chlor im molaren Verhältnis 1:1,1:0,1 gasförmig zugeführt und mit einer Lampenleistung von 700 W bei einer Reaktorinnentemperatur von 90 °C durch Pyrex-Glas (Borosilikat-Glas) mit λ > 280 nm bestrahlt. Die Edukte wurden hierbei kontinuierlich von oben dem Reaktor zugeführt und die Produkte am unteren Ausgang des Reaktors in eine Methanol/Trockeneis-Kühlfalle abgeführt. Die Dosierung betrug 3,3 mol Trichlorethylen in der Stunde. Die GC-Analyse der Kondensationsprodukte ergab Dichloracetylchlorid in einer Selektivität von 91 %. Weiterhin entstanden 2 % Trichlorethylenepoxid. Der Umsatz an Trichlorethylen betrug 83 %. Chlorierungsprodukte des Trichlorethylens und auch von DCAC wurden erstaunlicherweise nicht gefunden. Das Produkt wurde durch Destillation weiter gereinigt.

### Beispiel 3:

### Kontinuierliche Herstellung von Dichloracetylchlorid (DCAC) durch Photooxidation von Trichlorethylen durch Borosilikat-Glas und Cl₂-Sensibilisierung in der Gasphase bei 90 °C mit verringerter Lampenleistung

Durchführung wie in Beispiel 2, jedoch mit einer Lampenleistung von 500 W bei gleicher Dosierung. Die Selektivitäten betrugen für DCAC 92 % und für Trichlorethylenepoxid 2 %. Der Umsatz an Trichlorethylen lag bei 69 %.

### Beispiel 4:

### Kontinuierliche Herstellung von Dichloracetylchlorid (DCAC) durch Photooxidation von Trichlorethylen durch Borosilikat-Glas und Cl₂-Sensibilisierung in der Gasphase bei 90 °C bei höherer Dosierung

Durchführung wie Beispiel 2, jedoch mit einer Lampenleistung von 500 W und einem Trichlorethylen/Sauerstoff/Chlorverhältnis von 1:1,1:0,06 und einer Trichlorethylen-Dosierung von 3,52 mol/h. Die Selektivitäten betrugen für DCAC 96 % und für Trichlorethylenepoxid 1,6 %. Der Umsatz an Trichlorethylen lag bei 42 %.

### Beispiel 5:

### Kontinuierliche Darstellung von Dichloracetylchlorid (DCAC) durch Photooxidation von Trichlorethylen durch Pyrex-Glas und Cl₂-Sensibilisierung in der Gasphase bei 90 °C unter Verwendung dotierter Strahler

Durchführung wie Beispiel 4, jedoch mit einem mit Thalliumjodid dotiertem Hg-Strahler (Bezeichnung TQ 718 Z2, Firma Heraeus Noblelight). Die Selektivitäten entsprachen dem Beispiel 4, der Umsatz betrug jedoch 64 %.

### Beispiel 6 (Vergleichsbeispiel):

### Kontinuierliche Herstellung von Trichloracetylchlorid durch Photooxidation von Tetrachlorethylen durch Bestrahlung durch Pyrexglas und Cl₂-Sensibilisierung in der Gasphase bei 150 °C.

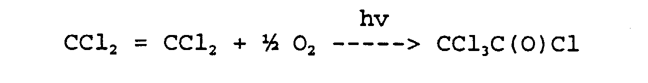

Einem 400 ml Photolyse Tauchschachtreaktor (außen mit Doppelmantel für die Heizung) ausgestattet mit einem nicht dotierten Hg-Hochdruckstrahler (Bezeichnung: TQ 718, Firma Heraeus Noblelight) wurde eine Mischung aus Tetrachlorethylen (aus Vorverdampfer mit T = 170 °C), Sauerstoff und Chlor im molaren Verhältnis 1:1,1:0,1 gasförmig zugeführt und mit einer Lampenleistung von 500 W bei einer Reaktorinnentemperatur von 150 °C durch Pyrex-Glas (Bosilikat-Glas) mit λ > 280 nm bestrahlt. Die Edukte wurden hierbei kontinuierlich von oben dem Reaktor zugeführt und die Produkte am unteren Ausgang des Reaktors in eine Methanol/Trockeneis-Kühlfalle abgeführt. Die Dosierung betrug 2,7 mol Tetrachlorethylen in der Stunde. Die GC-Analyse der Kondensationsprodukte ergab Trichloracetylchlorid in einer Selektivität von 96 %. Der Umsatz betrug 56 %. (Angabe der %-Zahlen jeweils in GC-Flächenprozent).

Das Arbeiten in Apparaturen aus Duran⁵⁰-Glas führte zu vergleichbaren Ergebnissen.

### Beispiel 7:

### Kontinuierliche Herstellung von DCAC durch Photooxidation von Trichlorethylen durch Bestrahlung durch Quarzglas ohne Chlor in der Gasphase bei 90 °C.

In einem 400 ml Photoreaktor mit einem nicht dotierten Hg-Hochdruckstrahler (Bezeichnung: TQ 718, Firma Heraeus Noblelight) wurde eine Mischung aus Trichlorethylen (aus Vorverdampfer mit T = 150 °C) und Sauerstoff im molaren Verhältnis 1:1,1 gasförmig zugeführt und mit einer Lampenleistung von 500 W bei einer Reaktorinnentemperatur von 90 °C durch Quarz-Glas bestrahlt. Die Edukte wurden hierbei kontinuierlich von oben dem Reaktor zugeführt und die Produkte am unteren Ausgang des Reaktors in eine Methanol/Trockeneis-Kühlfalle abgeführt. Die Dosierung betrug 2,2 mol Trichlorethylen in der Stunde. Die GC-Analyse der Kondensationsprodukte ergab Dichloracetylchlorid in einer Selektivität von 83 %. Weiterhin entstanden 3,5 % Trichlorethylenepoxid, 11 % COCl₂ und 2,8 % Oxalsäuredichlorid. Der Umsatz an Trichlorethylen betrug 63 %.

### Beispiel 8:

### Kontinuierliche Herstellung von DCAC durch Photooxidation von Trichlorethylen durch Bestrahlung durch Quarzglas ohne Chlor in der Gasphase bei 150 °C.

In einem 400 ml Photoreaktor mit einem nicht dotierten Hg-Hochdruckstrahler (Bezeichnung: TQ 718, Firma Heraeus Noblelight) wurde eine Mischung aus Trichlorethylen (aus Vorverdampfer mit T = 150 °C) und Sauerstoff im molaren Verhältnis 1:1,1 gasförmig von unten zugeführt und mit einer Lampenleistung von 700 W bei einer Reaktortemperatur von 150 °C durch Quarz-Glas bestrahlt. Die Dosierung betrug 2,2 mol Trichlorethylen in der Stunde. Der den Reaktor oben verlassende gasförmige Produktstrom enthielt DCAC in einer Selektivität von 67,4 %. Weiterhin entstanden die Nebenkomponenten Trichlorethylenepoxid (5,7 %), COCl₂ (17,2 %), Tetrachlorethylen (1,0 %), Pentachlorethyn (1,9 %), Hexachlorethan (0,3 %), Oxalsäuredichlorid (2,6 %) und Trichloracetylchlorid (3,1 %). Der Umsatz an Trichlorethylen betrug 48,3 %.

### Beispiele 9 und 10:

### Herstellung von Chlordifluoracetylchlorid mit Leuchtstofflampen als UV-Lichtquelle

**Allgemeines:** Verwendet wurde ein 4,3 Liter Volumen umfassender Photoreaktor aus Duran 50^{R}. Die Bestrahlung erfolgte mit einer UV-Lampe TLK4010R. Es handelt sich dabei um eine 40 Watt Nennleistung aufweisende, mit einer Reflektorschicht zur Erhöhung der Strahlungsleistung ausgestattete handelsübliche UV-Leuchtstofflampe der Firma Philips.

Sauerstoff, Chlor und 1,1-Dichlor-2,2-difluorethylen (1112a) wurden vor dem Reaktor gemischt und gasförmig in den Reaktor eingeleitet.

### Analytik:

Die Umsatzbestimmung erfolgt über die komplette Hydrolyse des Reaktorabgases, wonach mit Hilfe eines Ionenchromatographen der Gehalt an Chlordifluoressigsäure bestimmt wird. Die Selektivität wurde gaschromatographisch (Gasprobe nach Reaktor, vor Hydrolyse) ermittelt.

### Beispiel 9:

### Herstellung von Chlordifluoracetylchlorid (CDFAC)

Ansatz: 0,6 mol 1112a, 1,0 mol O₂ und 0,11 mol Cl₂.
Versuchsdauer: 20 min

1112a wird verdampft und mit den anderen Reaktionskomponenten gemischt durch den Reaktor geleitet. Hinter dem Reaktor befindet sich eine Waschflasche, um das entstandene Chlordifluoracetylchlorid zu hydrolysieren. Die Reaktortemperatur betrug dabei 85 bis 91 °C, bestrahlt wurde mit 2 Lampen ä 40 Watt. Der Umsatz betrug 99,58 %, die Selektivität bezogen auf entstandenes CDFAC lag bei 90,8 %.

### Beispiel 10:

Ansatz: 0,57 mol 1112a, 0,61 mol O₂ und 0,04 mol Cl₂
Versuchsdauer: 20 min

Die Durchführung erfolgt wie in Beispiel 9, mit der Ausnahme, daß mit nur einer Leuchtstofflampe, d. h. 1 x 40 Watt bestrahlt wird. Der Umsatz betrug 95,4 %, die Selektivität bezogen auf entstandenes CDFAC lag bei 90,26 %.

## Patentansprüche

1. Verfahren zur Herstellung von in α-Stellung durch mindestens 1 Chloratom substituierten Carbonsäurechloriden, nämlich Dichloracetylchlorid durch photochemische Oxidation von CCl₂ = CHCl oder CHCl₂CHCl₂ oder Difluorchloracetylchlorid durch photochemische Oxidation von CCl₂ = CF₂ als C2-Reaktant, mit Sauerstoff als Reaktant in der Gasphase, wobei man den C2-Reaktanten dampfförmig in den Reaktor einbringt, man das Verfahren kontinuierlich durchführt, man in Anwesenheit von elementarem Chlor arbeitet, wobei man eine aktivierende Bestrahlung mit Licht einer Wellenlänge von λ ≥ 280 nm vornimmt und man die Reaktanten in der Gasphase miteinander umsetzt.

2. Verfahren nach Anspruch 1, dadurch gekennzeichnet, daß man zur Herstellung von Dichloracetylchlorid von CCl₂ = CHCl ausgeht.

3. Verfahren nach Anspruch 1, dadurch gekennzeichnet, daß man drucklos arbeitet.

4. Verfahren nach einem der vorhergehenden Ansprüche, dadurch gekennzeichnet, daß das Molverhältnis zwischen dem C2-Reaktant und elementarem Chlor im Bereich von 1:0,01 bis 1:1, vorzugsweise im Bereich von 1:0,08 bis 1:0,2 liegt.

5. Verfahren nach einem der vorhergehenden Ansprüche, dadurch gekennzeichnet, daß das Molverhältnis zwischen dem C2-Reaktanten und Sauerstoff (O₂) im Bereich von 1:0,3 bis 1:5, vorzugsweise im Bereich von 1:0,4 bis 1:1,8 liegt.

6. Verfahren nach Anspruch 1, dadurch gekennzeichnet, daß man die Umsetzung bei einer Temperatur von bis zu 200 °C, vorzugsweise in einem Temperaturbereich von 50 bis 150 °C durchführt.

7. Verfahren nach Anspruch 6, dadurch gekennzeichnet, daß man die Umsetzung in einem Temperaturbereich durchführt, der zwischen dem Siedepunkt des C2-Reaktanten und des C2-Reaktionsprodukts liegt.

8. Verfahren nach Anspruch 7, dadurch gekennzeichnet, daß man das C2-Reaktionsprodukt flüssig vom Reaktorboden abzieht.

9. Verfahren nach einem der Ansprüche 1 bis 8, dadurch gekennzeichnet, daß die durchschnittliche Verweilzeit im Reaktor zwischen 0,01 und 30 Minuten, vorzugsweise zwischen 0,5 Minuten und 3,0 Minuten liegt.

10. Verfahren nach einem der vorhergehenden Ansprüche, dadurch gekennzeichnet, daß man zur aktivierenden Bestrahlung einen dotierten Strahler, vorzugsweise einen mit einem Metalljodid dotierten Hg-Hochdruckstrahler einsetzt.

11. Verfahren nach Anspruch 10, dadurch gekennzeichnet, daß man einen mit Galliumjodid, Kaliumjodid oder Cadmiumjodid dotierten Hg-Hochdruckstrahler einsetzt.

12. Verfahren nach Anspruch 1, dadurch gekennzeichnet, daß man in Abwesenheit eines die Umlagerung von intermediären Produkten wie Epoxiden beschleunigenden basischen Katalysators arbeitet.

## Claims

1. A process for the production of carboxylic acid chlorides substituted in the α position by at least one chlorine atom, namely dichloracetyl chloride, by photochemical oxidation of CCl₂=CHCl or CHCl₂CHCl₂ or difluorochloroacetyl chloride by photochemical oxidation of CCl₂=CF₂ as C2 reactant, with oxygen as reactant in the gas phase, wherein the C2 reactants are introduced into the reactor in vapour form, the process is performed continuously, it is carried out in the presence of elemental chlorine, with activating irradiation with light of a wavelength of λ ≥ 280 nm being carried out and the reactants being reacted together in the gas phase.

2. A process according to Claim 1, characterised in that CCl₂=CHCl is the starting point for the production of dichloroacetyl chloride.

3. A process according to Claim 1, characterised in that unpressurised operation is used.

4. A process according to one of the preceding claims, characterised in that the molar ratio between the C2 reactant and elemental chlorine lies in the range from 1:0.01 to 1:1, preferably in the range from 1:0.08 to 1:0.2.

5. A process according to one of the preceding claims, characterised in that the molar ratio between the C2 reactant and oxygen (O₂) lies in the range from 1:0.3 to 1:5, preferably in the range from 1:0.4 to 1:1.8.

6. A process according to Claim 1, characterised in that the reaction is performed at a temperature of up to 200°C, preferably in a temperature range from 50 to 150°C.

7. A process according to Claim 6, characterised in that the reaction is performed in a temperature range which lies between the boiling point of the C2 reactant and of the C2 reaction product.

8. A process according to Claim 7, characterised in that the C2 reaction product is withdrawn in liquid state from the bottom of the reactor.

9. A process according to one of Claims 1 to 8, characterised in that the average dwell time in the reactor is between 0.01 and 30 minutes, preferably between 0.5 minutes and 3.0 minutes.

10. A process according to one of the preceding claims, characterised in that a doped radiator, preferably an Hg high-pressure radiator doped with a metal iodide, is used for the activating irradiation.

11. A process according to Claim 10, characterized in that an Hg high-pressure radiator doped with gallium iodide, potassium iodide or cadmium iodide is used.

12. A process according to Claim 1, characterised in that the process is performed in the absence of a basic catalyst accelerating the rearrangement of intermediate products such as epoxides.

## Revendications

1. Procédé de préparation de chlorures d'acide carboxylique substitués en position α par au moins un atome de chlore, à savoir du chlorure de dichloracétyle par oxydation photochimique de CCl₂ = CHCl ou CHCl₂CHCl₂, ou du chlorure de difluorochloracétyle par oxydation photochimique de CCl₂ = CF₂ en tant que réactif en C₂, avec de l'oxygène en tant que réactif de la phase gazeuse, dans lequel on introduit le réactif en C₂ sous forme de vapeur dans le réacteur, on réalise le procédé en continu, on travaille en présence de chlore élémentaire, et dans lequel on procède à une irradiation activante avec de la lumière présentant une longueur d'onde λ ≥ 280 nm et on convertit les uns avec les autres les réactifs de la phase gazeuse.

2. Procédé selon la revendication 1, caractérisé en ce que l'on part de CCl₂ = CHCl pour fabriquer le chlorure de dichloracétyle.

3. Procédé selon la revendication 1, caractérisé en ce que l'on travaille sans pression.

4. Procédé selon l'une des revendications précédentes, caractérisé en ce que le rapport molaire entre le réactif en C₂ et le chlore élémentaire est compris dans la gamme allant de 1:0,01 à 1:1, de préférence dans la gamme allant de 1:0,08 à 1:0,2.

5. Procédé selon l'une des revendications précédentes, caractérisé en ce que le rapport molaire entre le réactif en C₂ et l'oxygène (O₂) est compris dans la gamme allant de 1:0,3 à 1:5, de préférence dans la gamme allant de 1:0,4 à 1:1,8.

6. Procédé selon la revendication 1, caractérisé en ce que la conversion est réalisée à une température allant jusqu'à 200°C, de préférence comprise dans une gamme de températures allant de 50 à 150 °C.

7. Procédé selon la revendication 6, caractérisé en ce que la conversion est réalisée dans une gamme de températures comprises entre le point d'ébullition du réactif en C₂ et celui du produit de la réaction en C₂.

8. Procédé selon la revendication 7, caractérisé en ce que le produit de la réaction en C₂ est évacué du fond du réacteur de façon fluide.

9. Procédé selon l'une des revendications 1 à 8, caractérisé en ce que le temps de séjour moyen dans le réacteur est compris entre 0,01 et 30 minutes, de préférence entre 0,5 minute et 3,0 minutes.

10. Procédé selon l'une des revendications précédentes, caractérisé en ce que l'on utilise, pour l'irradiation activante, un émetteur dopé, de préférence un émetteur haute pression au mercure dopé avec un iodure de métal.

11. Procédé selon la revendication 10, caractérisé en ce que l'on utilise un émetteur haute pression au mercure dopé avec de l'iodure de gallium, de l'iodure de potassium ou de l'iodure de cadmium.

12. Procédé selon la revendication 1, caractérisé en ce que l'on travaille en l'absence d'un catalyseur basique accélérant la conversion des produits intermédiaires comme des époxydes.
